# FASCICULE DE BREVET EUROPEEN

(11) **EP 4 346 616 B1**
(45) Date de publication et mention de la délivrance du brevet: **22.07.2026**
(21) Numéro de dépôt: 22730934.1
(22) Date de dépôt: 03.06.2022
(51) Int. Cl.: A61B 8/08, A61B 8/00

(54) **SYSTEME PORTATIF D'IMAGERIE**
TRAGBARES BILDGEBUNGSSYSTEM
PORTABLE IMAGING SYSTEM

(30) Priorité: 04.06.2021 EP 21305759
(43) Date de publication de la demande: 10.04.2024
(73) Titulaire: SORBONNE UNIVERSITE, 75006 Paris (FR); Centre National de la Recherche Scientifique, 75016 Paris (FR); INSERM (Institut National de la Santé et de la Recherche Médicale), 75013 Paris (FR)
(72) Inventeur: AISSANI, Abderrahmanne, 75019 Paris (FR); CHAVIGNON, Arthur, 94250 Gentilly (FR); COUDERT, Antoine, 75015 Paris (FR); COUTURE, Olivier, 92130 Issy-les-Moulineaux (FR); HEILES, Baptiste, 2563CG Den Haag (NL); HINGOT, Vincent, 75005 Paris (FR)
(74) Mandataire: Icosa
(86) Numéro de dépôt international: PCT/EP2022/065259
(87) Numéro de publication internationale: WO 2022/254037

(56) Documents cités:
- WO-A1-2018/104350
- WO-A2-2008/017997

## Description

### DOMAINE DE l'INVENTION

La présente invention concerne le domaine de l'imagerie médicale ultrasonore.

### ÉTAT DE LA TECHNIQUE

De manière bien connue en soi, l'échographie consiste à émettre et recevoir des ondes ultrasonores à travers les tissus et structures biologiques d'un patient et à exploiter les échos obtenus dans ces tissus et structures biologiques pour en reconstruire une image ultrasonore. Le terme d'image vaut pour toutes les représentations obtenues à partir de traitements des échos et peut aussi bien être 1D (sous forme, par exemple, d'un tableau de valeurs ou d'un score), 2D que 3D. L'échographie permet, par exemple, de mesurer l'échogénicité des tissus. Une image ultrasonore peut ainsi être une succession d'informations telle que des positions d'éléments de suivi.

Par ailleurs, l'introduction d'un élément de suivi, par exemple un agent de contraste, permet de rehausser les échos, donc le signal perçu, de tous les vaisseaux et ainsi obtenir une image de la perfusion des tissus. Un agent de contraste bien connu de l'état de la technique peut prendre la forme d'une solution de microbulles injectable en intraveineuse. En général, on injecte une série de microbulles à la fois. Typiquement, plusieurs dizaines ou centaines de millions dans le réseau vasculaire humain.

Classiquement, l'échographie étant basée sur une approche ondulatoire, elle est limitée par la diffraction, c'est-à-dire que la longueur d'onde utilisée limite la précision à laquelle les tissus et structures biologiques peuvent être caractérisés. Ainsi, de manière paradoxale, les courtes longueurs d'onde, favorables à une plus grande résolution, sont atténuées plus fortement dans les tissus que les longueurs d'ondes plus longues

L'art antérieur pertinent est divulgué dans les publications de brevet WO 2018/104350 A1 et WO 2008/017997 A2.

### OBJECTIF DE L'INVENTION

L'échographie super-résolue ou Ultrasound Localization microscopy abrégé par l'acronyme anglais « ULM » ou l'acronyme français « MLU » pour microscopie par localisation ultrasonore (Couture et al. IEEE UFFC 2018) est une technique qui contourne les règles de la diffraction à l'aide des microbulles mentionnées plus haut. En réalisant de nombreuses images d'un tissu contenant des vaisseaux dans lesquels circulent des microbulles, on peut déterminer la position de chacune des microbulles avec une résolution plus précise que la longueur d'onde. En accumulant la position super-résolue de chacune des microbulles, on peut créer une image des vaisseaux dont la résolution dépasse la limite théorique de diffraction. Jusque récemment, les échographes utilisaient très peu de post traitement et les étapes importantes de traitement de signal étaient réalisées de manière analogique. Depuis peu, les composants informatiques permettent de traiter des gros volumes de données simultanément et de travailler sur des signaux numérisés et envoyés vers un ordinateur. La super résolution ultrasonore (ULM) est une méthode de formation d'images exclusivement numérique permettant d'améliorer la résolution d'un facteur 10 par rapport aux méthodes conventionnelles. Elle s'appuie sur différentes étapes réalisées séquentiellement à l'aide d'un appareil permettant l'enregistrement des échos ultrasonores et leur traitement par un ordinateur. Cette méthode est aujourd'hui réalisée de manière séquentielle avec les étapes suivantes :
1) une séquence d'émission/réception des signaux ultrasonores définie par un logiciel : on parle de signaux RadioFréquence (RF), et ils correspondent à N enregistrements temporels réalisés par N capteurs ultrasonores. Chaque séquence d'émission/réception est répétée à haute cadence (par exemple 500 Hz),
2) une étape de formation de voie (ou beamforming) permettant de convertir ces signaux temporels en une image ultrasonore, plus précisément, une image ultrasonore caractérisant le milieu,
3) une étape de séparation des échos des microbulles des échos provenant des autres tissus,
4) une étape de détection permettant d'identifier chaque microbulle individuellement,
5) une étape de localisation sub-pixel consistant à déterminer la position d'une microbulle dans l'image avec une précision inférieure aux dimensions originales de l'image,
6) une étape de tracking, qui relie les positions d'une même microbulle entre des images successives pour former des trajectoires du parcours des microbulles dans la circulation sanguine,
7) une étape de traitement des trajectoires permettant de corriger des artefacts d'échantillonnages spatiaux et temporels,
8) une étape d'affichage, permettant de reconstruire l'intégralité de la vascularisation présente dans les champs de vue,
9) une étape de correction d'aberration des échos ultrasonores qui sont déformés lors de leur passage à travers un os (par exemple l'os du crane), qui peut être réalisée en parallèle ou en amont des étapes de traitement.
10) une étape de correction de mouvement qui peut être réalisée en parallèle des étapes de traitement et qui estime les déplacements entre les images successives de manière à les corriger.

La dimension de l'image ultrasonore dépend de la géométrie de la sonde utilisée. De manière bien connue en soi, une image 3D peut être obtenue en utilisant des sondes dites matricielles dont les capteurs sont disposés selon un plan.

L'imagerie médicale est une aide essentielle à l'établissement de nombreux diagnostics, en particulier les diagnostics liés aux AVC. Cependant, il arrive régulièrement, dans des cas d'urgence, que l'imagerie nécessaire au diagnostic ne puisse être effectuée assez rapidement soit parce que le temps de transport du patient est trop long, soit parce que la machinerie d'imagerie du centre de soins était déjà utilisée pour l'établissement d'un autre diagnostic, sur un autre patient. Le point clé du problème rencontré actuellement par l'imagerie médicale, est ainsi le manque de possibilités de diagnostic hors de l'hôpital. Or, il a été identifié que techniquement, cela est limité par les insuffisances des techniques d'imagerie et notamment des techniques conventionnelles à base d'ultrasons. Il y a donc un réel besoin d'un outillage d'imagerie médicale portatif que l'on peut amener vers le patient, sans avoir forcément besoin, dans un premier temps, d'amener le patient vers l'outillage d'imagerie médicale. Toutefois, indépendamment du fait de pouvoir ou non, créer une image médicale indépendamment du lieu où se trouve un patient en ayant potentiellement besoin, il faut ensuite pouvoir la lire et l'interpréter et ceci peut être impossible si aucun praticien expérimenté ne se trouve à proximité. Il y a donc un réel besoin d'un système permettant de créer de l'imagerie médicale pouvant être interprétée et lue par un praticien expérimenté indépendamment du lieu où se trouve le patient et le praticien

La présente invention a notamment pour objectif de proposer d'exploiter la technologie ultrasonore décrite ci-dessus permettant une super résolution de type ULM, dans le cadre de la prise en charge de patients ayant besoin d'un diagnostic par une telle technologie indépendamment du lieu où se trouve le patient et le praticien devant établir le diagnostic issu de cette technologie. Plus précisément, la présente invention a pour objectif de fournir de nouveaux moyens d'aide à la réalisation de diagnostic en mobilité (notamment par l'obtention d'images). L'obtention de nouveaux moyens facilitant la réalisation de diagnostics en portabilité est permise par la mise en œuvre de l'échographie super-résolue (ULM) et de ce que l'ULM permet de mettre en œuvre les différentes parties de l'invention décrite ci-dessous.

### EXPOSÉ DE L'INVENTION

On parvient à réaliser cet objectif au moyen d'un système d'imagerie ULM portatif destiné à visualiser une structure biologique cible d'un patient au moyen d'au moins un élément de suivi introduit à l'intérieur de ladite structure biologique cible, le système d'imagerie ULM portatif comprenant :
- une unité de contrôle avec une mémoire, la mémoire étant destinée à stocker au moins une image ultrasonore caractérisant la structure biologique cible,
- au moins une première sonde destinée à être fixée de manière amovible à une structure biologique de fixation du patient, la structure biologique de fixation entourant au moins partiellement la structure biologique cible, l'au moins première sonde étant reliée à l'unité de contrôle,
- un module d'aide au positionnement de l'au moins une sonde, le module d'aide au positionnement étant un module électronique actif,

l'au moins première sonde permettant au système de détecter l'au moins un élément de suivi au moyen d'ultrasons, l'unité de contrôle étant configurée pour localiser, en temps réel, l'au moins un élément de suivi à l'intérieur la structure biologique cible, de manière à mettre en œuvre un procédé d'échographie super-résolue dite ULM pour caractériser la structure biologique cible et créer l'image ultrasonore,
l'unité de contrôle étant en outre conçue pour afficher, sur un périphérique d'affichage (18), l'au moins une image ultrasonore stockée dans la mémoire,
l'image ultrasonore permettant à un opérateur d'établir un diagnostic rapide indépendamment de la localisation et de l'environnement du patient, caractérisé en ce que le module d'aide au positionnement calcule un indice de qualité de transmission pour chaque sonde.

Ainsi, grâce à l'ULM, cette solution permet d'atteindre l'objectif susmentionné. En particulier, toute personne nécessitant un diagnostic basé sur une imagerie médicale, peut être diagnostiqué indépendamment du lieu où elle se trouve et indépendamment du lieu où se trouve le praticien apte à établir le diagnostic. L'image créée, peu importe sa forme, a une forte valeur d'aide au diagnostic. Son utilisation en mobilité et par de non-experts est permise par les combinaisons et synergies entre l'échographie super-résolue (ULM) et les différents éléments du système selon l'invention qui permettent d'arriver à une bonne qualité diagnostic.

La mise en œuvre de l'ULM sur un tel système portable permet, grâce au gain en résolution qu'elle permet par rapport aux technologies conventionnelles de réaliser un diagnostic sur un système portable, là où les systèmes portables conventionnels en seraient incapables. Cela permet de plus d'embarquer plusieurs modules dont le fonctionnement est permis par l'ULM, et qui facilitent l'utilisation en mobilité et par des opérateurs non experts.

Le système selon l'invention peut comprendre une ou plusieurs des caractéristiques suivantes, prises isolément les unes des autres ou en combinaison les unes avec les autres :
- la structure biologique cible peut être le système sanguin cérébral et l'image ultrasonore peut être une angiographie,
- l'au moins un élément de suivi peut être un agent de contraste,
- le périphérique d'affichage peut être situé dans un lieu différent de l'unité de contrôle et/ou de l'au moins première sonde,
- l'unité de contrôle et le périphérique d'affichage peuvent communiquer par ondes électromagnétiques,
- le système peut comporter un module d'assistance au diagnostic,
- le module d'assistance au diagnostic peut permettre la mise en relation d'un premier opérateur au chevet du patient avec un deuxième opérateur en mesure de lire l'image ultrasonore affichée sur le périphérique d'affichage,
- le module d'assistance au diagnostic peut comporter un logiciel permettant d'assister un opérateur au chevet du patient,
- le module d'aide au positionnement peut être un module mécanique passif,
- le module d'aide au positionnement peut être un module électronique actif,
- le module d'aide au positionnement peut calculer un indice de qualité de transmission pour chaque sonde,
- le module d'aide au positionnement peut envoyer au moins un signal de rétroaction de manière à aider à maximiser l'indice de qualité,
- le système peut comprendre un module de transmission.

La présente invention a également pour objet un procédé d'imagerie rapide destiné à visualiser une structure biologique cible d'un patient, le procédé permettant la mise en œuvre du système selon l'une quelconque des caractéristiques ci-dessus, le procédé comportant les étapes suivantes, dans l'ordre chronologique d'énonciation :
a. branchement, si besoin, par un premier opérateur, de l'au-moins une sonde sur l'unité de contrôle,
b. engagement du module d'assistance au positionnement,
c. positionnement des sondes sur la structure biologique du patient,
d. acquisition d'une première image ultrasonore
e. correction, si nécessaire, du positionnement de l'au-moins une sonde au moyen du module d'assistance au positionnement, jusqu'à optimisation du signal perçu par l'unité de contrôle,
f. positionnement de l'au moins une sonde sur la structure biologique de fixation,
g. évaluation de la qualité des signaux avant lancement de la séquence d'acquisition d'une nouvelle image ultrasonore,
h. injection, en intraveineuse, dans les premières secondes de l'acquisition, d'éléments de suivi
   dans la structure biologique cible du patient, acquisition de l'image ultrasonore
i. correction, si nécessaire, du positionnement de l'au-moins une sonde au moyen du module d'assistance au positionnement, jusqu'à optimisation du signal perçu par l'unité de contrôle à partir de la technologie ULM,
j. création de l'image ultrasonore de la structure biologique cible par mise en œuvre de la technologie ULM,
k. transmission immédiate de l'image ultrasonore sur un périphérique d'affichage à portée d'un deuxième opérateur, caractérisé en ce que le module d'aide au positionnement (26) calcule, pour chaque sonde (24), un indice de qualité de transmission dans chaque étape de correction du positionnement.

Après l'étape de positionnement de l'au moins une sonde, le procédé peut comporter une étape de fixation de l'au moins une sonde sur la structure biologique de fixation.

### BRÈVE DESCRITPION DES FIGURES

L'invention sera mieux comprise, et d'autres buts, détails, caractéristiques et avantages de celle-ci apparaîtront plus clairement à la lecture de la description explicative détaillée qui va suivre, de modes de réalisation de l'invention donnés à titre d'exemples purement illustratifs et non limitatifs, en référence aux dessins schématiques annexés.

Sur ces dessins:
- la figure 1 est une illustration schématique d'un système d'imagerie portatif selon l'invention,
- la figure 2 est une illustration d'une image ultrasonore obtenue au moyen du système selon la présente invention,
- la figure 3a est une illustration schématique d'une sonde ultrasonore de type échographique utilisée dans le système selon l'invention,
- la figure 3b est une illustration schématique d'une sonde ultrasonore de type coudée utilisée dans le système selon l'invention.

### DESCRITPION DÉTAILLÉE

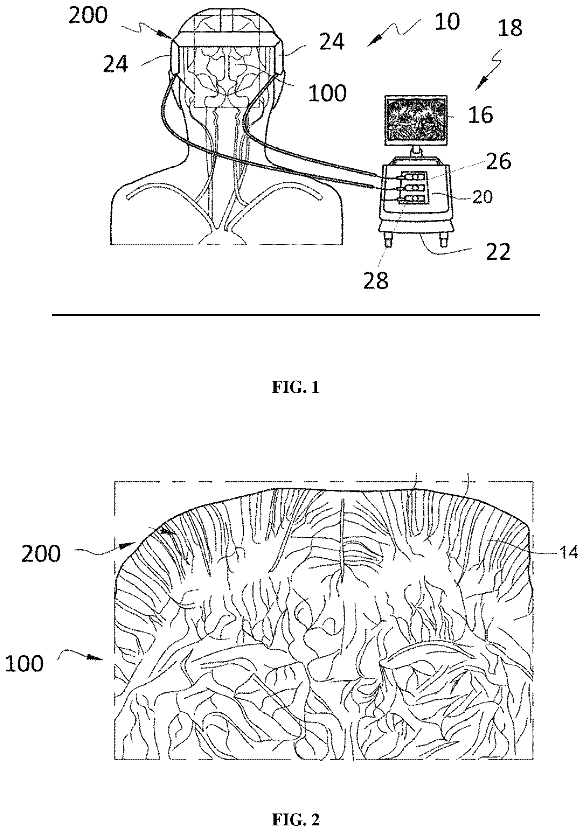

Comme visible sur la figure 1, la présente invention concerne un système portatif d'imagerie 10 destiné à visualiser une structure biologique cible 100 d'un patient, comme par exemple une vascularisation cérébrale, et à localiser un élément de suivi à l'intérieur de ladite structure biologique cible 100, de manière à pouvoir en créer une image ultrasonore 16 lisible sur un périphérique d'affichage 18, par exemple un écran.

Dans un mode de réalisation préférentiel, la structure biologique cible 100 est le système sanguin cérébral et l'image ultrasonore est une angiographie.

Selon un mode de réalisation particulier, l'image ultrasonore 16 est en 1D, par exemple sous la forme d'un tableau qui stocke les positions des éléments de suivi 14 (par exemple des microbulles) ou d'un score. L'image peut être aussi en 2D.

Dans un autre mode de réalisation, l'image ultrasonore 16 est en 3D. Elle permet d'acquérir un volume entier de la structure biologique cible 100 du patient à chaque injection d'éléments de suivi 14, plutôt que de reconstruire la structure biologique cible 100 plan-par-plan, ce qui nécessiterait de nombreuses injections d'éléments de suivi 14. Plus généralement, l'aspect 3D libère des questions de dépendance à l'opérateur. En effet, une imagerie 2D implique une sélection de plan par un opérateur alors qu'un volume 3D permet de tout acquérir et d'ensuite évaluer à posteriori. Ainsi, l'utilisation d'une image ultrasonore 3D offre une synergie supplémentaire à exploiter : l'utilisation de la super résolution (ULM) combinée à la 3D permet de limiter la dépendance à l'opérateur, et donc permet de fiabiliser le diagnostic en mobilité sans opérateur expert.

De manière alternative, le volume entier de la structure biologique 100 peut être reconstitué à partir d'une série de petits volumes 3D, partiels, de la structure biologique. Cette image ultrasonore 16 est destinée à permettre l'établissement rapide d'un diagnostic, en particulière dans le cas d'un accident vasculaire cérébral. Plus particulièrement, l'image ultrasonore 16 permet à un opérateur d'établir un diagnostic rapide indépendamment de la localisation et de l'environnement du patient. Il est ainsi possible de diagnostiquer un patient, chez lui, ou par exemple dans une ambulance. Dans la présente invention, la notion de « rapide » est définie par rapport à un trajet en ambulance : le diagnostic doit pouvoir être fait durant un trajet en ambulance. Le terme « rapide » réfère donc à une durée de plusieurs secondes à une vingtaine de minutes et doit être comparé aux durées actuelles de pose d'un diagnostic pouvant s'élever à plusieurs heures.

Ainsi, comme visible sur la figure 1, le système portatif d'imagerie 10 selon l'invention comporte :
- une unité de contrôle 20 avec une mémoire 22, ladite mémoire pouvant être localisée n'importe où dans le système 10,
- au moins une première sonde 24 destinée à être fixée de manière amovible à une structure biologique de fixation 200 du patient, la structure biologique de fixation 200 entourant au moins partiellement la structure biologique cible 100, l'au moins première sonde 24 étant reliée à l'unité de contrôle 20,
- un module d'aide au positionnement 26 de l'au moins une sonde 24.

Dans certains modes de réalisation, le système portatif d'imagerie portatif 10 selon le système 10 comprend un module d'assistance au diagnostic 28.

Par portatif on entend un système pouvant être déplacé par la simple force physique d'un ou plusieurs opérateur(s), sans nécessité d'outillage électronique ou d'outillage de transport type chariot à roulettes ou diable. Ainsi, par portatif on entend un système qui peut être déplacé facilement en étant porté par un utilisateur, par exemple au moyen de poignées ou de sangles. Idéalement la masse du système est comprise entre 10 et 30 kilogrammes.

Plus particulièrement, la portabilité (aspect portatif) du système 10 selon la présente invention est permise par l'implémentation de l'ULM ainsi que par les différentes synergies qu'offre l'ULM, c'est-à-dire :
- la télémédecine grâce à des données plus légères,
- la possibilité d'adapter le placement de la sonde automatiquement sur la base des données ULM,
- la possibilité de calculer des biomarqueurs spécifiques à partir des données ULM (voir plus loin dans la description pour des exemples précis).

C'est de la synergie entre les différentes parties de la présente invention que vient la portabilité du système 10 dans le sens ou son utilisation permet de réaliser un diagnostic partout et sans être dépendant d'un opérateur expert en technologie ULM.

Dans un mode de réalisation préféré, le système 10 présente un volume inférieur ou égale à 1m3 et un poids inférieur ou égal à 15kg.

L'élément de suivi est destiné à être introduit à l'intérieur de la structure biologique cible 100, par exemple par injection. Dans le cas où cet élément de suivi est un agent de contraste, plus particulièrement une microbulle, des millions d'éléments de suivi 14 sont ainsi destinés à être injectés dans le patient. Ils sont tous destinés à être localisés au sein de la structure biologique cible 100 au moyen de l'au-moins une sonde 24 et permettent ainsi de délimiter ladite structure biologique cible et en créer une image ultrasonore 16 exploitable en utilisant la technologie ULM.

### L'unité de contrôle

L'unité de contrôle 20 est destinée à produire et stocker au moins une image ultrasonore 16 de la structure biologique cible du patient. Elle comprend ainsi :
- un échographe numérique dont les principaux composants sont :
   o une carte d'acquisition et une carte d'émission électronique, ou une carte d'acquisition/émission électronique,
   o des composants informatiques (processeurs, carte graphique, etc...),
   o un boitier médical aux normes qui intègre les composants ci-dessus,
- un logiciel effectuant intégralement les taches suivantes :
   o interfaçage avec la carte électronique,
   o contrôle de l'émission et de la réception de signaux ultrasonores,
   o sauvegarde des signaux numérisés en mémoire,
   o formation de voies pour préparer les données à l'étape de traitement,
   o une étape logicielle de traitement permettant la super résolution ultrasonore,
   o l'interfaçage avec l'opérateur.

La mémoire 22, pouvant être située n'importe où dans le système 10, de l'unité de contrôle 20 est ainsi destinée à stocker l'au moins une image ultrasonore 16 de la structure biologique cible 100. Un exemple d'une telle image ultrasonore 16 2D est visible en figure 2.

L'unité de contrôle 20 est en outre conçue pour afficher, sur le périphérique d'affichage 18, l'au moins une image ultrasonore 16 stockée dans la mémoire 22. L'unité de contrôle 20 permet ainsi d'afficher (directement ou indirectement), la localisation de l'au moins un élément de suivi 14 dans ladite au moins une image ultrasonore 16.

L'unité de contrôle 20 comporte en outre un module de transmission, de manière à permettre la télétransmission des données collectées par les sondes 24 (données ULM, par exemple) pour permettre d'afficher l'image ultrasonore 16 sur un périphérique d'affichage 18 situé à un autre endroit que l'unité de contrôle 20, permettant ainsi à un autre opérateur d'établir un diagnostic à distance.

La présente invention cherchant à produire et présenter des moyens, notamment sous la forme d'une image ultrasonore, permettant l'établissement d'un diagnostic en mobilité en combinant l'échographie super-résolue (ULM) et la télémédecine, est, par définition liée au concept de télémédecine ambulatoire (urgente ou non) sur la base d'une imagerie rapide d'une structure biologique cible 100 d'un patient. En particulier, la présente invention permet une angiographie par ULM du cerveau et les données de placement, Doppler et ULM après leur traitement automatique peuvent ainsi, préférentiellement, être transmises à un opérateur (par exemple un radiologue) à distance. Cette transmission se faisant après le traitement informatique des données, par exemple le traitement ULM, la quantité de données à transmettre est fortement réduite et peut donc être transmise rapidement et aisément, permettant la mobilité. En effet, un volume entier de données ultrasonore ultrarapide 3D pour quelques minutes d'acquisition peut être de l'ordre du Teraoctet. La position de chacun des éléments de suivi 14 (par exemple des microbulles), même s'ils se comptent par millions, est une donnée très parcimonieuse qui peut être transmise plus facilement (Megaoctets). Suite à l'acquisition au chevet du patient, chaque image ultrasonore 16 vectorisée est transmise de façon sécurisée à distance au deuxième opérateur qui peut ensuite établir un diagnostic. Le diagnostic permet finalement d'établir si un traitement (par exemple une thrombolyse) doit être réalisé sur place. Les données brutes sont sauvegardées dans la mémoire 22 de l'unité de contrôle 20 en local, pour analyse et archivage, par exemple à l'arrivée à l'hôpital.

### L'au moins une première sonde

Dans le mode de réalisation illustré en figure 1, le système 10 comprend deux sondes 24. Ce sont des sondes ultrasonores. Chaque sonde 24 comporte ainsi au moins un capteur ultrasonore. De manière générale et bien connue en soi, la conception des sondes ultrasonores est habituellement faite pour qu'elles puissent être tenues et manipulées aisément à la main. Cela impose une forme quasi cylindrique où les câbles transmettant les signaux électriques entre chaque sonde 24 et l'unité de contrôle 20 sont perpendiculaires au plan contenant l'au-moins un capteur ultrasonore. Chaque sonde 24 est ainsi capable de faire la conversion ultrason - électronique et ainsi de permettre au système 10 de détecter chaque élément de suivi au moyen d'ultrasons. Plus particulièrement, chaque sonde 24 est échantillonnée spatialement dans deux dimensions pour pouvoir reconstruire un volume entier.

Chaque sonde 24, peut être une sonde matricielle, plus particulièrement, une sonde multiplexée. Dans ce cas, à minima, chaque sonde 24 doit être munie d'au moins plusieurs transducteurs dans deux dimensions. Dans certain cas, chaque sonde 23 peut être munie d'un quadrillage cartésien de transducteurs.

Ainsi, l'image ultrasonore 16 consiste en des données en trois dimensions obtenues grâce à une sonde 24 comprenant des transducteurs positionnés à minima sur deux dimensions (matrice d'éléments).

Comme chaque sonde 24 est reliée à l'unité de contrôle 20, l'unité de contrôle 20 peut ainsi localiser, en temps réel, chaque élément de suivi à l'intérieur la structure biologique cible 100. Ceci permet détailler et caractériser géométriquement la structure biologique cible 100 et permet de créer une image ultrasonore 16 de celle-ci.

Chaque sonde 24 peut être fixée sur la structure biologique de fixation 200 du patient au moyen, par exemple d'un casque (dans le cas où la structure biologique de fixation 200 est le crâne du patient) ou d'un collet muni d'une ou deux sondes 24, ou même tenu à la main. Dans un mode de réalisation alternatif, le patient peut être assis sur un siège où sont accrochées les sondes 24.

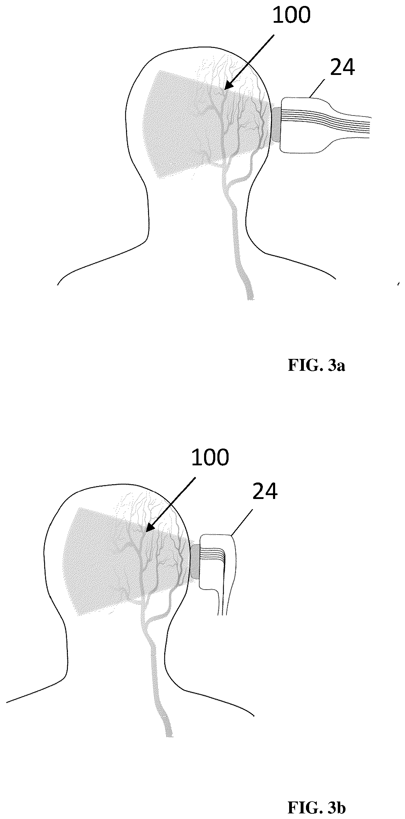

Chaque sonde 24 peut être conçue comme illustré en figure 3, c'est-à-dire avec les câbles positionnés dans le plan des capteurs ultrasonores. Ainsi, elle peut être tenue dans le creux de la main de l'opérateur, facilitant le maintien dans une position stable pendant un temps plus important.

Dans le cas où la structure biologique cible 100 est le système sanguin cérébral, la structure biologique de fixation 200 est le crâne du patient.

### Le module d'aide au positionnement de l'au moins une sonde

Le module d'aide au positionnement 26 est un système mécanique et/ou électronique de placement assisté de chaque sonde 24 sur la structure biologique de fixation 200.

L'échographie super-résolue se distingue de l'échographie standard par le fait qu'elle est effectuée en imageant le même endroit pendant de longues périodes. La période d'acquisition peut ainsi durer de quelques secondes à plusieurs minutes. En effet, pour collecter la position super-résolue de milliers d'élément de suivi tels que des agents de contraste, plus particulièrement des microbulles, tout en évitant que leurs signaux respectifs n'interfèrent, il est nécessaire de réaliser des milliers d'images de la même structure biologique cible 100 du patient. Par opposition, l'échographie standard est temps-réel et se réalise en balayant les organes avec une imagerie bi-dimensionnelle et en identifiant les plans d'intérêt au diagnostic.

Il est donc indispensable que chaque sonde 24 soit placée au contact de la structure biologique de fixation 200 de manière à ce que la zone d'émission de chaque faisceau ultrasonore émis par chaque sonde 24 permette d'obtenir un indice de qualité de transmission le plus élevé possible. Pour permettre au système 10 de fonctionner correctement, il faut donc s'assurer que chaque sonde 24 soit placée au bon endroit.

Le module d'aide au positionnement 26 vise ainsi à assister un opérateur non expert au bon positionnement de chaque sonde 24.

Dans un premier mode de réalisation, le module d'aide au positionnement 26 est un module mécanique passif, permettant un bon positionnement de chaque sonde 24 au moyen, par exemple, d'une complémentarité de forme avec une partie de l'anatomie du patient. Le module d'aide au placement peut ainsi comprendre une pièce ou une partie de pièce formant un négatif d'une partie anatomique d'un patient, par exemple l'oreille.

Selon l'invention, le module d'aide au positionnement 26 est un module électronique actif.

Ce mode de réalisation actif implique de faire des acquisitions pour caractériser la qualité de la transmission ultrasonore et identifier la position idéale dans laquelle l'émission ultrasonore doit être réalisée et donc la position idéale à laquelle chaque sonde 24 doit être fixée sur la structure biologique de fixation 200 du patient. La qualité de la transmission ultrasonore est déterminée à partir d'un indice de qualités calculé à partir d'un ou d'une combinaison des éléments suivants :
- l'imagerie ultrasonore basse résolution réalisée selon des modes d'imagerie déjà connus de l'état de l'art (par exemple l'imagerie Doppler pulsé) de la structure biologique cible 100,
- la reconnaissance du milieu imagé avec une sonde 2D,
- l'analyse de la distorsion des échos reçus par rapport à une propagation dans un milieu idéal, par exemple en mesurant l'écart à la réponse impulsionnelle spatiale ou la fonction de cohérence de l'écho ultrasonore, la distorsion étant une mesure réalisée à l'aide des éléments de suivi 14 et pouvant être directement calculée sur les données ULM
- l'analyse des signaux diffusés sur l'imagerie ultrasonore basse résolution réalisée selon des modes d'imagerie déjà connus de l'état de l'art, de propriétés intrinsèques de la structure biologique de fixation 200 (par exemple l'épaisseur de l'os du crane).

La zone d'émission de chaque faisceau ultrasonore est ensuite déterminée pour que l'indice de qualité de la transmission soit le plus élevé possible. L'orientation du faisceau peut se faire à partir d'une ou d'une combinaison des méthodes suivantes :
- par un mouvement physique de chaque sonde 24 pouvant être réalisé à la main par l'opérateur grâce à des indicateurs sensoriels visuels et/ou auditifs,
- de manière motorisée et/ou automatisée à l'aide d'un dispositif mécanique,
- de manière électronique et/ou automatisée à l'aide d'un dispositif acoustique permettant d'orienter le faisceau ultrasonore par contrôle électronique de l'émission.

Par « automatisé », on entend un déplacement électronique en changeant les phases ou les éléments de chaque sonde 24 utilisé. Il pourrait par exemple s'agir d'une très grosse sonde 24 avec beaucoup d'éléments dont seulement une fraction sont utilisés pour reconstruire l'image finale. Le positionnement serait ainsi l'étape pour établir la meilleure partie de la sonde 24 à utiliser.

Ainsi, une fois qu'un premier indice de qualité est calculé par le module d'aide au positionnement 26 pour un premier positionnement des sondes 24, un signal de rétroaction est envoyé par le module d'aide au positionnement 26 en indiquant la qualité du premier positionnement de chaque sonde 24. Si la qualité de ce premier positionnement n'est pas assez bonne, le module d'aide au positionnement indique également une direction de déplacement de la sonde 24, pour améliorer l'indice de qualité. Il peut s'agir d'un signal graduel afin de permettre un positionnement très précis. Le signal peut également prendre en compte l'historique du déplacement de chaque sonde 24. La sonde 24 est alors déplacée soit à la main, soit automatiquement et un prochain indice de qualité est calculé. Ceci est répété jusqu'à ce que l'indice de qualité soit satisfaisant.

Ces étapes de positionnement peuvent également être effectuées pendant l'imagerie pour éviter la dérive spatiale de la sonde échographique.

Le module d'aide au positionnement 26 de l'au moins une sonde 24 peut faire partie de l'unité de contrôle 20.

### Le module d'assistance au diagnostic

Selon un premier mode de réalisation, le module d'assistance au diagnostic 28 permet l'assistance d'un premier opérateur situé au chevet du patient, par un deuxième opérateur, préférentiellement un expert situé dans un lieu différent. Le module d'assistance au diagnostic 28 fonctionne grâce aux technologies de télécommunication et est, de même que le module de communication de l'unité de contrôle 20, lié au concept de télémédecine.

Selon un mode de réalisation alternatif, le module d'aide au diagnostic 28 utilise des biomarqueurs basés sur l'ULM. Plus précisément, le module d'aide au diagnostic peut calculer des biomarqueurs à partir des données ULM 16 afin d'affiner le diagnostic et d'assister un opérateur non expert. Des exemples de ces biomarqueurs sont proposés plus bas dans le texte.

Lors de l'utilisation du système 10 selon la présente invention, l'opérateur peut utiliser un module présent sur l'appareil et qui permet de réaliser les opérations suivantes :
- mise en relation de l'unité de contrôle 20 avec un expert non présent grâce à une ou plusieurs des méthodes de télécommunication suivante :
   o communication vocale entre le patient, l'opérateur et l'expert,
- communication visuelle entre le patient, l'opérateur et l'expert, contrôle à distance, par l'expert :
   o du logiciel,
   o du module d'aide au positionnement 26,
- échange de données d'intérêt, par exemple des méta données pertinents (position, environnement, etc...)
- échange informations médicales pertinentes et résultats d'examen vers et depuis l'unité de contrôle 20,
- aide à l'établissement d'un diagnostic à distance,
- proposition et/ou recommandation de traitement.

Dans les cas où le diagnostic permet d'établir l'éligibilité à un traitement d'urgence, le traitement peut être réalisé immédiatement par l'opérateur situé au chevet du patient, sur avis et en concertation continue avec l'expert.

Dans la présente demande, le terme « immédiat » fait référence à une durée plus courte que le trajet de retour vers l'hôpital, typiquement quelques minutes.

Selon un deuxième mode de réalisation, le module d'assistance au diagnostique 28 permet d'assister les opérateurs experts et non experts dans leur utilisation du système 10 selon l'invention au moyen d'un logiciel spécifique. Notamment en ce que les données ULM soient transmises de manière rapide par télémédecine. L'unité de contrôle 20 affiche alors, sur un périphérique d'affichage annexe, différents indicateurs permettant d'assister l'opérateur mais ne pose pas de diagnostic. Il prévoit plusieurs fonctionnalités :
- une aide au diagnostic différentiel,
- une analyse fine de l'image ultrasonore 16 obtenue.

Ce mode de réalisation peut être réalisé par exemple en utilisant un logiciel d'intelligence artificiel. Ainsi, c'est la combinaison entre les données issues de l'échographie super résolue (ULM) et le(s) procédé(s)/logiciel(s) d'intelligence artificielle qui permet de calculer des biomarqueurs spécifiques, permettant ainsi d'aider un opérateur non expert au diagnostic et par conséquence l'utilisation en mobilité du système 10 selon l'invention.

L'aide au diagnostic différentiel permet d'estimer la sévérité et la localisation de la zone à problème au sein de la structure biologique cible 100, par exemple un site de lésion. Cette fonction se base sur les pratiques médicales déjà mises en place et permet à l'opérateur de réaliser ces opérations directement sur l'appareil. Suivant les indications remplies par l'opérateur, le module d'assistance au diagnostic 28 suggère des probabilités de localisation de la zone à problème pour permettre un positionnement et une utilisation plus efficace.

Le module d'assistance au diagnostique 28 peut être couplé avec le module d'aide au positionnement 26 pour directement permettre de placer chaque sonde 24 de manière à imager une zone spécifique.

L'analyse fine de l'image obtenue par système 10 permet dans une ou des régions spécifiques de la structure biologique cible 100 du patient, de retraiter les données permettre l'extraction de paramètres quantitatifs. En particulier, le module d'assistance au diagnostique 28 peut déterminer, par exemple dans le cas d'une utilisation du système 10 selon la présente invention dans le cadre d'un patient présentant des signes d'AVC, des indicateurs médicaux de référence comme le Cerebral Blood Volume (CBV) ou Cerebral Blood Flow (CBF), qui sont des biomarqueurs classiques d'imagerie. Des biomarqueurs nouveaux seront également proposés, en particulier :
- la mesure de vitesse dans des vaisseaux individuels sélectionnés par l'opérateur,
- l'identification des différents territoires de la structure biologique cible 100, par exemple les différents territoires artériels et veineux,
- la topographie d'une zone particulière de la structure biologique cible 100, plus particulièrement la zone à problème et, plus précisément, dans le cas d'un AVC, de la zone d'hypoperfusion.

D'autres indications, autres que l'hypoperfusion, sont envisageables, entre autres des paramètres généraux sur les parcours d'éléments de suivi 14, tels que des histogrammes de vitesse, de direction, de dispersion.

En utilisant des procédés d'intelligence artificielle, des probabilités associées avec les différents diagnostics connus peuvent être estimées de manière à guider l'opérateur. En particulier, la comparaison de l'image ultrasonore 16 avec une banque de donnée pertinentes, par exemple des différents territoires artériels dans le cas d'un AVC, ou avec une imagerie contra-latérale de l'autre hémisphère du cerveau du patient, permet de quantifier la certitude avec laquelle le diagnostic (par exemple d'ischémie) peut être proposé.

Dans un contexte clinique d'urgence, pour le cas particulier d'un patient ayant subi un AVC, et avec un système 10 utilisant l'imagerie ULM, le système 10 selon l'invention peut être utilisé, peu importe l'environnement dans lequel se trouve le patient, de la façon suivante :
- appel aux services urgences et arrivée des secours équipés du système d'imagerie d'urgence 10 en fonction des symptômes décrits et du résultat de l'anamnèse du patient pour lequel les secours ont été appelés,
- établissement d'un diagnostic préliminaire reposant sur l'examen de syndromes neurologiques (atteinte visuelle, aphasie, hémiparésie etc...), et de manifestation cliniques caractéristiques déjà utilisées dans le parcours de l'état de la technique,
- si un AVC est suspecté :
   o branchement d'une ou des sondes 24 sur l'unité de contrôle 20,
   o potentielle mise en place d'un ECG sur le patient et connecté à l'unité de contrôle 20,
   o engagement du module d'assistance au positionnement 26,
   ∘ application de gel sur les tempes du patient, positionnement des sondes 24 sur les tempes,
   o acquisition d'une première imagerie,
   o correction du positionnement des sondes 24 au moyen du module d'assistance au positionnement 26 , jusqu'à optimisation du signal perçu par l'unité de contrôle 20,
   o fixation des sondes 24 sur la tête du patient,
   o évaluation de la qualité des signaux avant lancement de la séquence d'acquisition ULM,
   o l'acquisition échographique ULM,
   o injection, dans les premières secondes de l'acquisition, d'éléments de suivi 14, plus particulièrement d'agents de contraste en intraveineuse,
   o potentielle correction du positionnement des sondes 24 avec les données ULM
   o création de l'image ultrasonore 16 de la structure biologique cible 100, plus particulièrement, d'une angiographie grâce à la technologie ULM,
   o transmission immédiate de l'image ultrasonore 16 vers un périphérique d'affichage 18, ledit périphérique d'affichage 18 étant à distance, la transmission permettant à un neuroradiologue, une personne entraînée à la lecture de ces ULM (un expert) ou un système expert de lire l'image ultrasonore 16,
   o établissement d'un diagnostic par un opérateur expert ou non (le neuroradiologue, la personne entraînée à la lecture de ces ULM ou le système expert, par exemple),
- si le diagnostic confirme la présence d'un thrombus, alors le médecin valide l'injection de thrombolytique au patient, réduisant significativement le temps de traitement,
- le patient est amené à l'hôpital pour un diagnostic plus poussé par IRM ou scanner, et est traité.

La présente invention propose donc un système de diagnostic en mobilité, issu de la combinaison de l'utilisation d'un système d'imagerie portatif 10 avec un procédé d'échographie super-résolue (ULM). Ce sont, précisément, les synergies entre l'ULM et différents éléments du système qui forment la clé au diagnostic en mobilité.

Éventuellement, plus tard, lorsque le patient est stabilisé, le système 10 selon la présente invention peut, par exemple, être utilisé pour les applications suivantes :
- monitoring régulier des vaisseaux et micro-vaisseaux sanguins pour déterminer l'évolution du cerveau du patient post-AVC permettant ainsi de prédire entre autres les évolutions négatives liées aux vasospasmes,
- suivi de patient, hors urgence, de manière générale.

## Revendications

1. Système d'imagerie ULM portatif (10) destiné à visualiser une structure biologique cible (100) d'un patient au moyen d'au moins un élément de suivi (14) introduit à l'intérieur de ladite structure biologique cible (100), le système d'imagerie ULM portatif (10) comprenant :
- une unité de contrôle (20) avec une mémoire (22), la mémoire (22) étant destinée à stocker au moins une image ultrasonore (16) caractérisant la structure biologique cible (100),
- au moins une première sonde (24) destinée à être fixée de manière amovible à une structure biologique de fixation (200) du patient, la structure biologique de fixation (200) entourant au moins partiellement la structure biologique cible (100), l'au moins première sonde (24) étant reliée à l'unité de contrôle (20),
- un module d'aide au positionnement (26) de l'au moins une sonde (24), le module d'aide au positionnement (26) étant un module électronique actif,
l'au moins première sonde (24) permettant au système (10) de détecter l'au moins un élément de suivi (14) au moyen d'ultrasons, l'unité de contrôle (20) étant configurée pour localiser, en temps réel, l'au moins un élément de suivi (14) à l'intérieur la structure biologique cible (100), de manière à mettre en œuvre un procédé d'échographie super-résolue dite ULM pour caractériser la structure biologique cible (100) et créer l'image ultrasonore (16),
l'unité de contrôle (20) étant en outre conçue pour afficher, sur un périphérique d'affichage (18), l'au moins une image ultrasonore (16) stockée dans la mémoire (22), l'image ultrasonore (16) permettant à un opérateur d'établir un diagnostic rapide indépendamment de la localisation et de l'environnement du patient,
**caractérisé en ce que** le module d'aide au positionnement (26) calcule un indice de qualité de transmission pour chaque sonde (24).

2. Système (10) selon la revendication précédente, **caractérisé en ce que** la structure biologique cible (100) est le système sanguin cérébral et l'image ultrasonore (16) est une angiographie.

3. Système (10) selon l'une quelconque des revendications précédentes, **caractérisé en ce que** l'au moins un élément de suivi (14) est un agent de contraste.

4. Système (10) selon l'une quelconque des revendications précédentes, **caractérisé en ce que** le périphérique d'affichage (18) est situé dans un lieu différent de l'unité de contrôle (20) et/ou de l'au moins première sonde (24).

5. Système (10) selon l'une quelconque des revendications précédentes, **caractérisé en ce que** l'unité de contrôle (20) et le périphérique d'affichage (18) communiquent par ondes électromagnétiques.

6. Système (10) selon l'une quelconque des revendications précédentes, **caractérisé en ce que** le système (10) comporte un module d'assistance au diagnostic (28).

7. Système (10) selon la revendication précédente, **caractérisé en ce que** le module d'assistance au diagnostic (28) permet la mise en relation d'un premier opérateur au chevet du patient avec un deuxième opérateur en mesure de lire l'image ultrasonore (16) affichée sur le périphérique d'affichage (18).

8. Système (10) selon l'une quelconque des revendications 6 ou 7, **caractérisé en ce que** le module d'assistance au diagnostic (28) comporte un logiciel permettant d'assister un opérateur au chevet du patient.

9. Système (10) selon l'une quelconque des revendications précédentes, **caractérisé en ce que** le module d'aide au positionnement (26) envoie au moins un signal de rétroaction de manière à aider à maximiser l'indice de qualité.

10. Système (10) selon l'une quelconque des revendications précédentes, **caractérisé en ce que** le système (10) comprend un module de transmission.

11. Procédé d'imagerie rapide destiné à visualiser une structure biologique cible d'un patient, le procédé permettant la mise en œuvre du système (10) selon l'une quelconque des revendications précédentes, le procédé comportant les étapes suivantes, dans l'ordre chronologique d'énonciation :
a. branchement, si besoin, par un premier opérateur, de l'au-moins une sonde (24) sur l'unité de contrôle (20),
b. engagement du module d'assistance au positionnement (26),
c. positionnement des sondes (24) sur la structure biologique fixation (200) du patient,
d. acquisition d'une première image ultrasonore (16),
e. correction, si nécessaire, du positionnement de l'au-moins une sonde (24) au moyen du module d'assistance au positionnement (26), jusqu'à optimisation du signal perçu par l'unité de contrôle (20),
f. positionnement de l'au moins une sonde (24) sur la structure biologique de fixation (200),
g. évaluation de la qualité des signaux avant lancement de la séquence d'acquisition d'une nouvelle image ultrasonore (16),
h. injection, en intraveineuse, dans les premières secondes de l'acquisition, d'éléments de suivi (14) dans la structure biologique cible (100) du patient,
i. acquisition de l'image ultrasonore (16),
j. correction, si nécessaire, du positionnement de l'au-moins une sonde (24) au moyen du module d'assistance au positionnement (26) jusqu'à optimisation du signal perçu par l'unité de contrôle (20) à partir de la technologie ULM,
k. création de l'image ultrasonore (16) de la structure biologique cible (100) par la mise en œuvre de la technologie ULM,
l. transmission immédiate de l'image ultrasonore (16) sur un périphérique d'affichage (18) à portée d'un deuxième opérateur, **caractérisé en ce que** le module d'aide au positionnement (26) calcule, pour chaque sonde (24), un indice de qualité de transmission dans chaque étape de correction du positionnement.

## Patentansprüche

1. Tragbares ULM-Bildgebungssystem (10), das dazu bestimmt ist, eine biologische Zielstruktur (100) eines Patienten mittels mindestens eines in die biologischen Zielstruktur (100) eingeführten Trackingelements (14) zu visualisieren, wobei das tragbare ULM-Bildgebungssystem (10) umfasst:
- eine Steuereinheit (20) mit einem Speicher (22), wobei der Speicher (22) dazu bestimmt ist, mindestens ein Ultraschallbild (16) zu speichern, das die biologische Zielstruktur (100) charakterisiert,
- mindestens eine erste Sonde (24), die dazu bestimmt ist, lösbar an einer biologischen Befestigungsstruktur (200) des Patienten befestigt zu werden, wobei die biologische Befestigungsstruktur (200) die biologische Zielstruktur (100) mindestens teilweise umgibt, wobei die mindestens erste Sonde (24) mit der Steuereinheit (20) verbunden ist,
- ein Positionierungshilfsmodul (26) der mindestens einen Sonde (24), wobei das Positionierungshilfsmodul (26) ein aktives elektronisches Modul ist,
wobei die mindestens erste Sonde (24) dem System (10) ermöglicht, das mindestens eine Trackingelement (14) mittels Ultraschalls zu erkennen, wobei die Steuereinheit (20) so eingerichtet ist, dass sie in Echtzeit das mindestens eine Trackingelement (14) innerhalb der biologischen Zielstruktur (100) lokalisiert, um ein superaufgelöstes Ultraschallverfahren, das als ULM bezeichnet wird, durchzuführen, um die biologische Zielstruktur (100) zu charakterisieren und das Ultraschallbild (16) zu erzeugen,
wobei die Steuereinheit (20) ferner ausgebildet ist, um auf einem Anzeigeperipheriegerät (18) das mindestens eine im Speicher (22) gespeicherte Ultraschallbild (16) anzuzeigen,
wobei es das Ultraschallbild (16) einem Bediener ermöglicht, unabhängig vom Standort und von der Umgebung des Patienten eine schnelle Diagnose zu stellen, **dadurch gekennzeichnet, dass** das Positionierungshilfsmodul (26) für jede Sonde (24) einen Übertragungsqualitätsindex berechnet.

2. System (10) nach dem vorhergehenden Anspruch, **dadurch gekennzeichnet, dass** die biologische Zielstruktur (100) das zerebrale Blutsystem ist und das Ultraschallbild (16) eine Angiographie ist.

3. System (10) nach einem der vorhergehenden Ansprüche, **dadurch gekennzeichnet, dass** das mindestens eine Trackingelement (14) ein Kontrastmittel ist.

4. System (10) nach einem der vorhergehenden Ansprüche, **dadurch gekennzeichnet, dass** sich das Anzeigeperipheriegerät (18) an einem anderen Ort als die Steuereinheit (20) und/oder die mindestens erste Sonde (24) befindet.

5. System (10) nach einem der vorhergehenden Ansprüche, **dadurch gekennzeichnet, dass** die Steuereinheit (20) und das Anzeigeperipheriegerät (18) über elektromagnetische Wellen kommunizieren.

6. System (10) nach einem der vorhergehenden Ansprüche, **dadurch gekennzeichnet, dass** das System (10) ein Diagnoseassistenzmodul (28) aufweist.

7. System (10) nach dem vorhergehenden Anspruch, **dadurch gekennzeichnet, dass** das Diagnoseassistenzmodul (28) die Verbindung eines ersten Bedieners am Patientenbett mit einem zweiten Bediener ermöglicht, der in der Lage ist, das auf dem Anzeigeperipheriegerät (18) angezeigte Ultraschallbild (16) zu lesen.

8. System (10) nach einem der Ansprüche 6 oder 7, **dadurch gekennzeichnet, dass** das Diagnoseassistenzmodul (28) eine Software aufweist, die es ermöglicht, einen Bediener am Patientenbett zu unterstützen.

9. System (10) nach einem der vorhergehenden Ansprüche, **dadurch gekennzeichnet, dass** das Positionierungshilfsmodul (26) mindestens ein Rückmeldesignal sendet, um die Maximierung des Qualitätsindex zu unterstützen.

10. System (10) nach einem der vorhergehenden Ansprüche, **dadurch gekennzeichnet, dass** das System (10) ein Übertragungsmodul umfasst.

11. Schnellbildgebungsverfahren, das dazu bestimmt ist, eine biologische Zielstruktur eines Patienten zu visualisieren, wobei das Verfahren die Umsetzung des Systems (10) nach einem der vorhergehenden Ansprüche ermöglicht, wobei das Verfahren die folgenden Schritte in der chronologischen Reihenfolge der Nennung umfasst:
a. erforderlichenfalls Anschließen, durch einen ersten Bediener, der mindestens einen Sonde (24) an die Steuereinheit (20),
b. Einsetzen des Positionierhilfsmoduls (26),
c. Positionieren der Sonden (24) auf der biologischen Befestigungsstruktur (200) des Patienten,
d. Erfassen eines ersten Ultraschallbilds (16),
e. gegebenenfalls Korrigieren der Positionierung der mindestens einen Sonde (24) mittels des Positionierungshilfsmoduls (26), bis das von der Steuereinheit (20) wahrgenommene Signal optimiert ist,
f. Positionieren der mindestens einen Sonde (24) auf der biologischen Befestigungsstruktur (200),
g. Bewerten der Qualität der Signale vor dem Starten der Erfassungssequenz eines neuen Ultraschallbilds (16),
h. intravenöses Injizieren, in den ersten Sekunden der Erfassung, von Trackingelementen (14) in die biologische Zielstruktur (100) des Patienten,
i. Erfassen des Ultraschallbilds (16),
e. gegebenenfalls Korrigieren der Positionierung der mindestens einen Sonde (24) mittels des Positionierungshilfsmoduls (26), bis das von der Steuereinheit (20) wahrgenommene Signal ausgehend von der ULM-Technologie optimiert ist,
k. Erzeugen des Ultraschallbilds (16) von der biologischen Zielstruktur (100) durch die Anwendung der ULM-Technologie,
I. sofortiges Übertragen des Ultraschallbilds (16) auf ein Anzeigeperipheriegerät (18) in Reichweite eines zweiten Bedieners, **dadurch gekennzeichnet, dass** das Positionierungshilfsmodul (26) für jede Sonde (24) in jedem Schritt der Positionierungskorrektur einen Übertragungsqualitätsindex berechnet.

## Claims

1. Portable ULM imaging system (10) intended to visualize a target biological structure (100) of a patient by means of at least one tracking element (14) introduced inside said target biological structure (100), the portable ULM imaging system (10) comprising:
• a control unit (20) with memory (22), the memory (22) being intended to store at least one ultrasound image (16) characterizing the target biological structure (100),
• at least a first probe (24) intended to be fixably attached in a removable manner to a fixation biological structure (200) of the patient, the fixation biological structure (200) surrounding at least partially the target biological structure (100), said at least first probe (24) being connected to the control unit (20),
• a positioning assistance module (26) for said probe(s) (24), the positioning assistance module (26) being an active electronic module,
said at least first probe (24) enabling the system (10) to detect said at least one tracking element (14) by means of ultrasound, the control unit (20) being configured to locate, in real time, said at least one tracking element (14) inside the target biological structure (100), so as to implement a super-resolved ultrasound procedure known as ULM to characterize the target biological structure (100) and create the ultrasound image (16),
the control unit (20) being further designed to display, on a display device (18), said at least one stored ultrasound image (16),
the ultrasound image (16) enabling an operator to establish a rapid diagnosis independently of the location and environment of the patient,
**characterized in that** the positioning assistance module (26) calculates a transmission quality index for each probe (24).

2. System (10) according to the preceding claim, **characterized in that** the target biological structure (100) is the cerebral blood system and the ultrasound image (16) is an angiography.

3. System (10) according to any one of the preceding claims, **characterized in that** said at least one tracking element (14) is a contrast agent.

4. System (10) according to any one of the preceding claims, **characterized in that** the display device (18) is located in a place different from the control unit (20) and/or the at least first probe (24).

5. System (10) according to any one of the preceding claims, **characterized in that** the control unit (20) and the display device (18) communicate via electromagnetic waves.

6. System (10) according to any one of the preceding claims, **characterized in that** the system (10) comprises a diagnostic assistance module (28).

7. System (10) according to the preceding claim, **characterized in that** the diagnostic assistance module (28) enables the linking of a first operator at the patient's bedside with a second operator capable of reading the ultrasound image (16) displayed on the display device (18).

8. System (10) according to any one of claims 6 or 7, **characterized in that** the diagnostic assistance module (28) comprises software to assist an operator at the patient's bedside.

9. System (10) according to any one of the preceding claims, **characterized in that** the positioning assistance module (26) sends at least one feedback signal so as to help maximize the quality index.

10. System (10) according to any one of the preceding claims, **characterized in that** the system (10) comprises a transmission module.

11. Rapid imaging method intended to visualize a target biological structure of a patient, the method enabling the implementation of the system (10) according to any one of the preceding claims, the method comprising the following steps, in chronological order of enumeration:
a. connection, if necessary, by a first operator, of said at least one probe (24) to the control unit (20),
b. engagement of the positioning assistance module (26),
c. positioning of the probes (24) on the patient's fixation biological structure (200),
d. acquisition of a first ultrasound image (16),
e. correction, if necessary, of the positioning of said at least one probe (24) by means of the positioning assistance module (26), until optimization of the signal perceived by the control unit (20),
f. positioning of the at least one probe (24) on the fixation biological structure (200),
g. evaluation of the signal quality before launching the acquisition sequence of a new ultrasound image (16),
h. intravenous injection, within the first few seconds of the acquisition, of tracking elements (14) into the target biological structure (100) of the patient,
i. acquisition of the ultrasound image (16),
j. correction, if necessary, of the positioning of said at least one probe (24) by means of the positioning assistance module (26) until optimization of the signal perceived by the control unit (20) starting from ULM technology,
k. creation of the ultrasound image (16) of the target biological structure (100) by implementing the ULM technology,
l. immediate transmission of the ultrasound image (16) to a display device (18) within reach of a second operator, **characterized in that** the positioning assistance module (26) calculates, for each probe (24), a transmission quality index at each step of the positioning correction
